# FASCICULE DE BREVET EUROPEEN

(11) **EP 1 334 714 B1**
(45) Date de publication et mention de la délivrance du brevet: **12.09.2007**
(21) Numéro de dépôt: 03290275.1
(22) Date de dépôt: 04.02.2003
(51) Int. Cl.: A61K 8/67, A61Q 19/00, A61Q 19/08

(54) **Utilisation d'acide ascorbique pour renforcer la cohésion de la jonction dermo-épidermique**
Verwendung von Ascorbinsäure zur Verstärkung der dermo-epidermischen Bindung
Use of ascorbic acid to stergthen the dermal-epidermal junction

(30) Priorité: 07.02.2002 FR 0201510
(43) Date de publication de la demande: 13.08.2003
(73) Titulaire: L'ORÉAL, 75008 Paris (FR)
(72) Inventeur: Bernerd, Francoise, 75018 Paris (FR)
(74) Mandataire: Allab, Myriam

(56) Documents cités:
- EP-A- 0 738 508
- WO-A-02/15860
- WO-A-96/25143
- WO-A-99/24009
- FR-A- 2 612 775
- FR-A- 2 779 059
- US-B1- 6 403 108
- DATABASE CAPLUS [en ligne] CHEMICAL ABSTRACTS SERVICE, COLUMBUS, OHIO, US; POTDAR ET AL.: "modulation by vitamin C of tumor incidence and inhibition in oral carcinogenesis" retrieved from STN Database accession no. 1993:471437 XP002217104
- DATABASE MEDLINE [en ligne] MICHEL ET AL.: "characterization of a new tissue-engineered human skin equivalent with hair" Database accession no. NLM10476918 XP002217105
- DATABASE MEDLINE [en ligne] YAMAMOTO ET AL.: "collagen synthesis in human skin fibroblasts is stimulated by a stable form of ascorbate, 2-O-alpha-D-glucopyranosyl-L-ascorbic acid" Database accession no. NLM1552361 XP002217106

## Description

La présente invention se rapporte à l'utilisation dans un milieu cosmétiquement acceptable, de l'acide ascorbique ou l'un de ses analogues pour renforcer la cohésion de la jonction dermo-épidermique.

L'acide ascorbique (ou vitamine C) est couramment utilisé dans les compositions topiques car il est connu qu'il a un effet bénéfique sur la peau. En particulier, il stimule la synthèse du tissu conjonctif, renforce les défenses du tissu cutané contre les agressions extérieures telles que les rayonnements ultraviolets et la pollution, compense la déficience en vitamine E de la peau, dépigmente la peau et possède une fonction anti-radicaux libres.

Une autre activité de la vitamine C décrite dans la littérature est son action sur le fibroblaste. Des travaux ont montré que la vitamine C permet d'augmenter la production de collagène de type I et III, avec une action sur différents niveaux de régulation. La vitamine C a notamment une activité post-traductionnelle, en interagissant sur les enzymes de maturation des collagènes I et III comme co-facteur pour la prolylhydroxylase.
Des études plus récentes ont aussi porté sur l'effet de la vitamine C au niveau transcriptionnel. La vitamine C régule ainsi la transcription du gène du collagène I (Slack JL, Liska DJ, Bornstein P. Regulation of the expression of the type I collagen genes. Am. J. Med. Genet. 1993, 45(2) : 140-51) et des ARNm du collagène III dans des fibroblastes de peau (Geesin JC, Darr D, Kaufman R, Murad S, Pinnell SR. Ascorbic acid specifically increases type I and type III procollagen mesRNA levels in human skin fibrobalst. J. Invest. Dermatol. 1988, 90(4) :420-4). En revanche, il semble que l'acide ascorbique n'ait que peu d'effet sur la transcription du gène du collagène de type IV (Tajima and Pinnell, 1996). Enfin des travaux plus récents ont montré que l'addition de vitamine C dans les cultures d'épiderme reconstruit avait une influence sur la fonction barrière en améliorant le profil lipidique des cellules épidermiques (Ponec M, Weerheim A, Kempenaar J, Mulder A, Gooris GS, Bouwstra J, Mommaas AM. The formation of competent barrier lipids in reconstructed human epidermis requires the presence of vitamin C. J. Invest. Dermatol. 1997, 109(3) :348-55).

La peau humaine est constituée de deux tissus l'un superficiel, l'épiderme, l'autre profond, le derme.
L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac, ...), appelé "fonction barrière".
Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.

La cohésion entre l'épiderme et le derme est assurée par la jonction dermo-épidermique. C'est une région complexe d'une épaisseur de 100 nm environ qui comprend le pôle basal des kératinocytes basaux, la membrane épidermique et la zone sous basale du derme superficiel (Bernard P. Structure de la jonction dermo-épidermique. Objectif peau. 2001, 68 : 87-93).
D'un point de vue structurel, des hémidesmosomes, dans lesquels s'insèrent des filaments de kératine (complexe hémidesmosome-tonofilaments), sont répartis sur la membrane plasmique des kératinocytes basaux.
Au regard de ces complexes hémidesmosome-tonofilaments, on trouve des filaments d'ancrage qui traversent la membrane basale épidermique. Les filaments d'ancrage sont reliées à la laminine 5 côté épidermique.
Enfin, des fibrilles d'ancrage constituent le réseau sous basal. Ce sont des structures curvilinéaires qui prennent naissance et se terminent sur la face profonde de la membrane basale et dans lesquelles viennent s'engager des fibres de collagène I, III et V.
Il a été montré que ces fibrilles d'ancrage, parfaitement visualisables en microscopie électronique, sont composées de collagène de type VII (ci-après collagène VII). Le collagène VII est synthétisé par les kératinocytes et les fibroblastes mais de façon plus importante par les kératinocytes (Aumailley M, Rousselle P. laminins of the dermo-epdiermal junction. Matrix Biology. 1999, 18: 19-28; Nievers M, Schaapveld R, Sonnenberg A. Biology and function of hemidesmosomes. Matrix Biology, 1999, 18 : 5-17).

Les collagènes sont les protéines majoritaires des matrices extracellulaires. A ce jour, 20 types de collagènes sont identifiés et notés de I à XX. On distingue différentes familles parmi ces types en fonction des structures formées :
- la famille des collagènes fibrillaires (type I, II, II V/XI) qui forment donc des fibres ;
- la famille des collagènes formant le réseau des membranes basales qui comprend le collagène de type IV et de type XVII ;
- les collagènes formant des réseaux hexagonaux (type VIII et type X), des filaments perlés (Type VI), des FACIT (type IX, XII, XIV, XVI, XIX, XX) ;
- les fibrilles d'ancrage qui correspondent au type VII ;
- les multiplexines (type XV, XVII) et
- le collagène de type XIII dont on ne connaît pas les fonctions précises à ce jour.

Dans la peau, les collagènes majoritairement présents dans l'ensemble du derme sont les collagènes de type I et III qui forment la matrice extracellulaire de l'ensemble du derme (ce sont ces collagènes qui constituent 70-80% du poids sec du derme).

Les collagènes représentent une famille d'une très grande diversité, en effet, chaque type de collagène et les chaînes qui le constituent est issu de gènes différents. Par exemple, les collagènes de type I sont composés de deux chaînes COL1A1 et COL1A2 dont les gènes sont localisés respectivement sur les chromosomes 17q et 7q. Le collagène de type VII est composé d'une seule chaîne et son gène est localisé sur les chromosome 3p. Par ailleurs, les collagènes ne sont pas tous synthétisés par les mêmes types cellulaires, les collagènes de type I et III sont essentiellement produits par le fibroblaste dermique alors que le collagène de type VII est produit par le kératinocyte épidermique. Enfin, la régulation de leur expression diffère d'un collagène à un autre, par exemple, les collagènes I et VII ne sont pas régulés de la même manière par certaines cytokines, en effet, le TNF alpha et la leukoreguline stimulent le collagène VII et régulent négativement le collagène I.

La cohésion entre les deux tissus, épiderme et derme, est essentielle pour l'intégrité de la peau dans son ensemble et permet une résistance aux agressions externes de type mécanique (frottements, coups etc..).

La jonction dermo-épidermique est également une structure qui conditionne l'état de surface de la peau.

Ainsi, une jonction dermo-épidermique présentant des structures d'ancrage intègres est maintenue plissée, permettant ainsi d'augmenter la surface de la zone de contact entre le derme et l'épiderme, de favoriser les échanges entre ces deux tissus, de renforcer leur cohésion et d'améliorer l'apparence de l'épiderme.
Dans des cas où les structures d'ancrage sont altérées (déficience de la synthèse de collagène VII ou de ténascine, vieillissement...), ceci provoque un aplanissement de la jonction dermo-épidermique. Les échanges sont amoindris, les deux tissus sont moins solidaires, l'épiderme se plisse et la peau étant moins ferme et moins tendue, les rides apparaissent et la fragilité de la peau aux agressions mécaniques est accrue.

L'absence ou la non fonctionnalité des composés de la jonction dermo-épidermique provoque des pathologies de type épidermolyse bulleuse. La zone de clivage est déterminée par la localisation de la protéine déficiente.
C'est notamment le cas pour des personnes présentant des mutations situées dans le gène codant pour le collagène VII. Ces mutations induisent des épidermolyses bulleuses (EB), telles que Hallopeau-Siemens type of récessive Dystrophic EB, Mild-type of récessive dystrophic EB, Dominat dystrophic EB. Ces maladies génétiques sont caractérisées par l'absence de formation de ces fibrilles d'ancrage, dont le résultat est une extrême fragilité de la peau avec décollement des deux tissus (derme - épiderme) aboutissant à la formation de « bulles ». D'autres épidermolyses bulleuses peuvent aussi avoir une origine auto-immune, avec production d 'auto-anticorps dirigés contre le collagène VII, comme l'epidermolysis bullosa acquisita, le bullus systemic lupus erythematosus et la maladie de Bowel. Dans tous les cas les epidermolyses bulleuses engendrent des anomalies qui sont très invalidantes car les personnes atteintes doivent être extrêmement prudentes et ne provoquer aucune tension ou traumatisme qui pourrait engendrer un effet mécanique au niveau de la jonction dermo-épidermique.

Akiyama *et al.* ont décrit par ailleurs que le collagène VII jouait aussi un rôle important au niveau du follicule pileux humain, notamment au niveau des membranes basales de la matrice (zone péripapillaire) et au niveau de la membrane du bulge (renflement de la partie haute du bulbe). Ces deux zones contiennent des cellules à fort potentiel mitotique, en particulier des kératinocytes donnant naissance à la tige pilaire. Or ces auteurs ont indiqué que le collagène VII présent dans le follicule est indispensable à l'expression de cette activité mitotique (Akiyama M, Dale BA, Sun TT, Holbrook KA. Characterisation of hair follicle bulge in human fetal skin: the human fetal bulge is a pool of undifferentiated keratinocytes. J. Invest. Dermatol. 1995, 105 : 844-50). Un autre constituant majeur de la jonction dermo-épidermique est la ténascine.
La ténascine est une glycoprotéine de la matrice extracellulaire, aussi appelée ténascine-C. Sa fonction essentielle réside dans les interactions épithélium-mésenchyme, notamment au cours de l'embryogénèse.
Dans la peau, on trouve de la ténascine au niveau sub-épidermique dans le derme papillaire, mais aussi autour des vaisseaux, et des annexes. Son expression est fortement augmentée dans les situations d'hyperprolifération comme le psoriasis, les tumeurs mais aussi dans la cicatrisation. La ténascine est produite dans la peau par les deux types cellulaires majeurs, le kératinocyte et le fibroblaste. Une de ses fonctions réside dans l'adhésion cellulaire. En effet, la forte régulation positive de la ténascine dans les kératinocytes en migration durant la cicatrisation laisse fortement supposer un rôle essentiel d'adhésion des kératinocytes sur le tissu conjonctif, assurant une bonne cohésion dermo-épidermique (Crossin KL. Tenascin: a multifunctional extracellular matrix protein with a restricted distribution in development and disease. J. Cell. Biochem. 1996, 61 : 592-598; Latijnhouwers M, Bergers M, Ponec M, Dijkman H, Andriessen M, Schlkwijk J. Human epidermal keratinocytes are a source of tenascin-C during wound healing. J. Invest Dermatol., 1997, 108 : 776-783 ; Steijlen PM, Maessen E, Kresse H, Van Vlijmen IMJJ, Verstraeten AA, Traupe H, Schalkwijk J. Expression of tenascin, biglycan and decorin in disorders of keratinization. Br. J. Dermatol., 1994, 130 : 564-568)

La Demanderesse a mis en évidence de façon surprenante que l'acide ascorbique (ou vitamine C) permettait d'augmenter la synthèse de collagène VII.

La vitamine C peut ainsi être utilisée dans des compositions cosmétiques pour renforcer la cohésion de la jonction dermo-épidermique du cuir chevelu.

A la connaissance de la Demanderesse, seuls certains rétinoïdes, notamment l'acide rétinoïque ou le rétinol (Woodley DT, Zelickson AS, Briggaman RA, Hamilton TA, Weiss JS, Ellis CN, Voorhees JJ. Treatment of photoaged skin with topical tretinoin increases epidermal-anchoring fibrils. A preliminary report. JAMA. 1990, 263(22) :3057-9), des extraits de la plante *Potentilla erecta* (brevet US 6,193,975) et des extraits de levure *Saccharomyces cerevisiae* (extrait de levure vendu sous le nom commercial Toniskin par Silab) permettaient à ce jour d'obtenir les mêmes effets. On connaît par ailleurs l'effet irritant des rétinoides sur la peau.

La demande de brevet EP 0 738 508 décrit une composition comprenant un ester d'ose de l'acide ascorbique et un filtre UVA sulfonique utile pour lutter contre les taches pigmentaires et le vieillissement, la vitamine C y est décrite comme un anti-oxydant et anti-radicalaire. La demande de brevet WO 02/15860 décrit quant à elle une composition topique antioxydante comprenant de la vitamine C décrite comme un composé anti-oxydant et anti-radicalaire favorisant l'action de la vitamine E et stimulant la synthèse de collagène I. L'objet de ces deux documents est de proposer des compositions dans lesquelles l'acide ascorbique présente une bonne stabilité.
Aucun document de l'état de la technique ne décrit l'effet bénéfique de la vitamine C sur la jonction dermo-épidermique, que ce soit par l'augmentation de la synthèse de ténascine ou par l'augmentation de la synthèse de collagène VII.

La présente invention a donc pour objet l'utilisation cosmétique de l'acide ascorbique ou de l'un de ses analogues dans une composition comprenant un milieu cosmétiquement acceptable pour augmenter la synthèse de collagène VII et la résistance de la peau aux agressions mécaniques choisies parmi frottements, tensions, frictions.

L'objet de l'invention est l'utilisation cosmétique dans un milieu cosmétiquement acceptable, de l'acide ascorbique ou l'un de ses analogues pour augmenter la synthèse collagène VII.

Grâce aux propriétés de ces protéines, collagène VII et ténascine, qui participent à l'attachement et à l'ancrage des kératinocytes épidermiques dans la matrice extracellulaire dermique, c'est l'ensemble de la cohésion dermo-épidermique qui est ainsi assurée. De plus, leur implication sur le plan mécanique augmente la résistance de la peau au stress mécanique, aux frottements, aux frictions. Cet aspect est notamment illustré par la formation de « bulles » ou « ampoules » dans les maladies (génétique ou autoimmunes citées plus haut) liées à une déficience en collagène VII. De façon plus courante, cet aspect est également illustré qu'une peau présentant des déficiences au niveau de la jonction dermo-épidermique est moins ferme et élastique.

Il a aussi été montré que la ténascine mais aussi la collagène VII étaient diminués ou régulés négativement par les glucocorticoïdes qui sont connus pour induire une fragilité de la peau. Augmenter le collagène VII permet donc de contrecarrer cette fragilité.
Il est aussi mentionné qu'une atrophie de la peau dans maladies liées à une déficience en collagène VII.

Ces protéines, collagène VII et ténascine, de la jonction dermo-épidermique ont aussi des fonctions de médiateurs de l'adhésion cellulaire, en particulier des cellules épidermiques, de médiateurs pour la migration des kératinocytes sur la matrice conjonctive, et participent à la transmission des signaux de la matrice extracellulaire vers l'épiderme (Burgeson RE and Christiano A.M. Current Opinion in cell Biology, 1997, 9 : 651-658).

La jonction dermo-épidermique joue donc un rôle d'interface dynamique entre le tissu conjonctif et l'épithélium. Elle gouverne l'intégrité structurale de l'organe peau dans son entier, et contrôle le passage de molécule entre le derme et l'épiderme.
Cette zone est essentielle lors des processus de remodelage ou de cicatrisation de la peau.

Ainsi, la composition cosmétique sera en particulier destinée à renforcer les structures d'ancrage entre le derme et l'épiderme et/ou améliorer la fixation et l'adhésion des cellules épidermiques au support matriciel et/ou augmenter la résistance de la peau aux agressions mécaniques telles que frottements, tensions, frictions.

Dans une variante particulière, l'invention se rapporte à l'utilisation cosmétique de l'acide ascorbique ou l'un de ses analogues pour toute application où l'on cherche à augmenter la synthèse de collagène VII.

Les compositions cosmétiques selon la présente invention pourront contenir une quantité efficace d'acide ascorbique ou de ses analogues, c'est-à-dire la quantité nécessaire pour obtenir les effets attendus selon l'invention. Pour donner un ordre de grandeur, cette quantité représente préférentiellement de 0,001% à 20% du poids total de la composition, préférentiellement de 0,1% à 15% du poids total de la composition et avantageusement de 1% à 10% du poids total de la composition.

En outre, la composition de l'invention sera également utilisée pendant un temps suffisant pour obtenir les effets attendus selon l'invention. Pour donner un ordre de grandeur, cette durée peut être au minimum de 15 jours, mais peut être aussi de plus de 4 semaines, voire de plus de 8 semaines.

Pour donner un ordre de grandeur, cette quantité représente préférentiellement de 0,001% à 20% du poids total de la composition, plus préférentiellement de 0,1% à 15% du poids total de la composition et avantageusement de 1% à 10% du poids total de la composition.

Pour la préparation des compositions cosmétiques de l'invention, on pourra utiliser l'acide ascorbique, qui est généralement sous forme L car il est le plus souvent extrait de produits naturels, ou ses analogues.

En raison de sa structure chimique (alpha-cétolactone) qui le rend très sensible à certains paramètres de l'environnement tels que la lumière, la chaleur et les milieux aqueux, il pourra être avantageux d'utiliser l'acide ascorbique sous la forme d'un ester d'ose de l'acide ascorbique ou d'un sel métallique d'acide ascorbique phosphorylé.

Les esters d'ose de l'acide ascorbique utilisables dans l'invention sont notamment les dérivés glycosylé, mannosylé, fructosylé, fucosylé, galactosylé N-acétylglucosaminé, N-acétylmuramique de l'acide ascorbique et leurs mélanges et plus spécialement l'ascorbyl-2 glucoside ou 2-O-α-D glucopyranosyl de l'acide L-ascorbique ou encore le 6-O-β-D galactopyranosyl de l'acide L-ascorbique. Ces derniers composés ainsi que leurs procédés de préparation, sont en particulier décrits dans les documents EP-A-487404, EP-A-425066 et JP05213736.

De son côté, le sel métallique d'acide ascorbique phosphorylé peut être choisi parmi les ascorbyl phosphates de métal alcalin, les ascorbyl phosphates de métal alcalino-terreux et les ascorbyl phosphates de métal de transition.

Les analogues de l'acide ascorbique sont, plus particulièrement, ses sels, tels que notamment l'ascorbate de sodium, l'ascorbylphosphate de magnésium ou de sodium, ses esters, tels que notamment ses esters acétique, propionique ou palmitique, ou ses sucres, tels que notamment l'acide ascorbique glycosylé.

Les compositions cosmétiques selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H) ou d'une émulsion triple (E/H/E ou H/E/H), de suspensions ou d'émulsions de consistance molle, semi-solide ou solide du type crème ou gel, de microémulsions, ou encore de microcapsules, de microparticules ou de dispersions vésiculaires de type ionique et/ou non ionique. Ces compositions sont préparées selon les méthodes usuelles bien connues de l'homme de l'art du domaine.

Selon une forme de réalisation particulière de l'invention, les compositions selon l'invention se présentent sous forme d'un support et d'un solide distincts, destinés à être mélangés extemporanément, le solide consistant essentiellement en de l'acide ascorbique pulvérulent. Un tel agencement est décrit par exemple dans la demande FR-94-03982, déposée par la Demanderesse et l'exemple 1 de la présente demande illustre ce type de formulation.

De façon connue, les compositions cosmétiques de l'invention peuvent contenir également les adjuvants habituels dans les domaines considérés, tels que les gélifiants hydrophiles ou lipophiles, les agents anti-glycation, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents tenseurs, les actifs lipolytiques ou ayant une activité favorable, directe ou indirecte, sur la diminution du tissu adipeux, les agents agissant sur la microcirculation, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées selon l'invention.

Les compositions de l'invention peuvent ainsi comprendre aussi des actifs, hydrophiles ou lipophiles, et notamment les actifs susceptibles de compléter l'effet de l'acide ascorbique.

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (Vaccinium angusfifollium) ; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 99/16166, FR 00/08158, FR 99/09267 et FR 99/16168, respectivement. Le resvératrol est particulièrement préféré pour une utilisation dans cette invention.

Parmi les actifs stimulant les macromolécules du derme, on peut citer ceux qui agissent :
- soit sur la synthèse du collagène, tels que les extraits de Centella asiatica ; les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®.} ; et les hormones végétales telles que les auxines.
- soit sur la synthèse d'élastine, tels que l'extrait de Saccharomyces cerivisiae commercialisé par la société LSN sous la dénomination commerciale Cytovitin^{®} ; et l'extrait d'algue Macrocystis pyrifera commercialisé par la société SECMA sous la dénomination commerciale Kelpadelie^{®} ;
- soit sur la synthèse des glycosaminoglycanes, tels que le produit de fermentation du lait par Lactobacillus vulgaris, commercialisé par la société BROOKS sous la dénomination commerciale Biomin yogourth^{®} ; l'extrait d'algue brune Padina pavonica commercialisé par la société ALBAN MÜLLER sous la dénomination commerciale HSP3^{®} ; et l'extrait de Saccharomyces cerevisiae disponible notamment auprès de la société SILAB sous la dénomination commerciale Firmalift^{®} ou auprès de la société LSN sous la dénomination commerciale Cytovitin^{®} ;
- soit sur la synthèse de la fibronectine, tels que l'extrait de zooplancton Salina commercialisé par la société SEPORGA sous la dénomination commerciale GP4G^{®}; l'extrait de levure disponible notamment auprès de la société ALBAN MÜLLER sous la dénomination commerciale Drieline^{®} ; et le palmitoyl pentapeptide commercialisé par la société SEDERMA sous la dénomination commerciale Matrixil^{®} ;
- soit sur l'inhibition métalloprotéinases (MMP) telles que plus particulièrement les MMP 1, 2, 3, 9 . On peut citer : les rétinoïdes et dérivés, les isoflavonoïdes, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge, de lin, de kakkon ou de sauge ;
- soit sur l'inhibition des sérine protéases telles que l'élastase leucocytaire ou la cathepsine G. On peut citer: l'extrait peptidique de graines de iégumineuse (Pisum sativum) commercialisé par la société LSN sous la dénomination commerciale Parelastyl^{®} ; les héparinoïdes ; et les pseudodipeptides.

Parmi les actifs stimulant les macromolécules épidermiques, telles que la fillagrine et les kératines, on peut citer notamment l'extrait de lupin commercialisé par la société SILAB sous la dénomination commerciale Structurine^{®} ; l'extrait de bourgeons de hêtre Fagus sylvatica commercialisé par la société GATTEFOSSE sous la dénomination commerciale Gatuline^{®} ; et l'extrait de zooplancton Salina commercialisé par la société SEPORGA sous la dénomination commerciale GP4G^{®}.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine^{®}; le beta-sitosteryl sulfate de sodium commercialisé par la société SEPORGA sous la dénomination commerciale Phytocohésine^{®} ; et l'extrait de maïs commercialisé par la société SOLABIA sous la dénomination commerciale Phytovityl^{®}.

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :
(1) les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les protéines végétales de soja ou de blé, et/ou
(3) les silicates de sodium et magnésium (Laponites).

Parmi les dérivés susceptibles de favoriser la lipolyse on peut trouver :
1) les inhibiteurs de phosphodiestérase, tels que :
   - les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ;
   - les associations contenant des dérivés xanthiques, comme l'association de caféine et de silanol (dérivé méthylsilanetriol de caféine), et par exemple le produit commercialisé par la société Exsymol sous la dénomination caféisilane C ;
   - les composés d'origine naturelle contenant des bases xanthiques et notamment de la caféine, tels que les extraits de thé, de café, de guarana, de maté, de cola (Cola Nitida) et notamment l'extrait sec de fruit de guarana (Paulina sorbilis) contenant 8 à 10 % de caféine ;
   - l'éphédrine et ses dérivés qui peuvent notamment se retrouver à l'état naturel dans les plantes telles que le Ma Huang (Ephedra plant) ;
2) les extraits végétaux et les extraits d'origine marine, qui sont soit actifs sur les récepteurs à inhiber, tels que les β-2-bloqueurs, les NPY-bloqueurs (décrits dans le brevet EP 838217), soit inhibent la synthèse des récepteurs aux LDL ou VLDL, soit actifs pour stimuler les récepteurs β et les protéines G, conduisant à l'activation de l'adénylcyclase. Comme extraits végétaux de ce type, on peut citer par exemple :
   - le Garcinia Cambogia,
   - les extraits de Bupleurum chinensis,
   - les extraits de lierre grimpant (Hedera Helix), d'arnica (Arnica Montana L), de romarin (Rosmarinus officinalis N), de souci (Calendula officinalis), de sauge (Salvia officinalis L), de ginseng (Panax ginseng), de millepertuis (Byperycum Perforatum), de fragon (Ruscus aculeatus L), d'ulmaire (Filipendula ulmaria L), d'orthosiphon (Orthosiphon Stamincus Benth), de bouleau (Betula alba), de cécropia et d'arganier,
   - les extraits de ginkgo biloba,
   - les extraits de prêle,
   - les extraits d'escine,
   - les extraits de cangzhu,
   - les extraits de chrysanthellum indicum,
   - les extraits de dioscorés riches en diosgénine ou la diosgénine ou hécogénine pure et leurs dérivés,
   - les extraits des plantes du genre Armeniacea, Atractylodis Platicodon, Sinom-menum, Pharbitidis, Flemingia,
   - les extraits de Coleus tels que C. Forskohlii, C. blumei, C. esquirolii, C. scutellaroïdes , C. xanthantus et C. Barbatus, tel que l'extrait de racine de Coleus Barbatus contenant 60 % de forskoline,
   - les extraits de Ballote,
   - les extraits de Guioa, de Davallia, de Terminalia, de Barringtonia, de Trema, d'Antirobia.
   - comme extrait d'origine marine on peut citer :les extraits d'algues ou de phytopancton, tels que le rhodystérol ou l'extrait de Laminaria Digitata commercialisé sous la dénomination PHYCOX75 par la société Secma, l'algue skeletonema décrite dans le brevet FR 2 782 921 ou les diatomées décrites dans le brevet FR 2774292 ;
3) les peptides ou protéines :
   - les peptides dérivés de l'hormone parathyroidienne tels que décrits dans les brevets FR 2 788058 et FR 2781231 de Séderma ou les peptides décrits dans le document FR 2 786 693 voire tout autre peptide ayant des propriétés lipolytiques,
   - les protamines et leurs dérivés tels que ceux décrits dans le document FR-A-2,758,724.

La quantité d'actif(s) lipolytique(s) peut varier dans une large mesure et dépend de la nature de ou des actifs utilisés. De manière générale, le ou les actifs amincissants sont présents en une concentration allant de 0,001 à 20 % et de préférence de 0,1 à 10 % en poids par rapport au poids total de la composition.

Comme actifs agissant sur la microcirculation (vasoprotecteur ou vasodilatateur), on peut citer les flavonoïdes, les ruscogénines, les esculosides, l'escine extraite du marron d'Inde, les nicotinates, l'héperidine méthyl chalcone, le petit houx, les huiles essentielles de lavande ou de romarin, les extraits de Ammi Visnaga.

Les actifs préférés selon la présente invention sont les extraits de maïs et de soja, les extraits d'algue Macrocystis pyrifera, le polysilicone-8, la caféine, les extraits de lierre et de Terminalia sericea. Ils sont particulièrement adaptés aux compositions destinées à redessiner le contour du visage.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles végétales (fraction liquide de beurre de karité), les huiles animales, les huiles de synthèse (huile de purcellin, polyisobutène hydrogénée), les huiles siliconées et les huiles fluorées (perfluoropolyéthers). On peut aussi utiliser comme matières grasses des alcools gras, des acides gras, des cires.

Comme émulsionnants utilisables dans l'invention, on peut citer par exemple les esters d'acide gras et de polyol, tels que les esters gras de sorbitol, comme le tristéarate de sorbitan vendu sous la dénomination de Span 65 par la société ICI, ou aussi les esters gras de glycérol tels que le monostéarate de glycérol, ou encore les esters de PEG tels que le stéarate de PEG-40 vendu sous la dénomination de Myrj 52 par la société ICI. Il peut s'agir aussi d'émulsionnants siliconés tels que le cétyl diméthicone copolyol vendu sous la dénomination d'Abil EM90 par la société Goldschmidt.

La Demanderesse a en effet observé que l'addition de vitamine C dans le milieu de culture d'une peau reconstruite pendant toute la phase d'émersion induit une augmentation très importante du nombre de fibroblastes dans la lattice. Cette augmentation est associée avec une production plus importante de certaines protéines de la matrice extracellulaire, en particulier dans la zone sous-épidermique. Les résultats les plus spectaculaires se situent au niveau de cette région de la jonction dermo-épidermique, avec un arrangement plus perpendiculaire des kératinocytes basaux et la synthèse augmentée de composants majeurs de la membrane basale, comme la ténascine et le collagène VII.

Cette utilisation permet ainsi d'obtenir une nette amélioration du modèle. Les peaux reconstruites obtenues sont plus proches de la peau humaine normale, en particulier, les phénomènes observés sur les peaux reconstruites sont plus prédictifs des phénomènes qui seront observés *in vivo.*

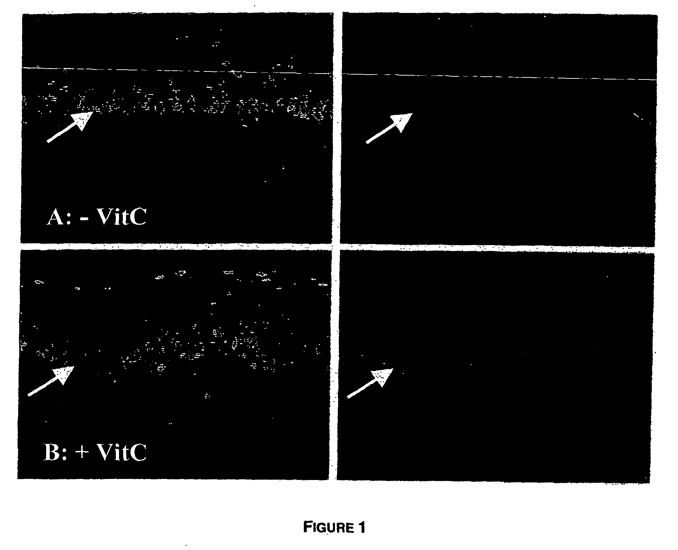

Cela ressort notamment des figures 1 à 3 annexées, commentées dans l'exemple 3 ci-dessous et dans lesquelles :
La **figure 1** représente des photographies de coupe transversale de peau reconstruite : pour les clichés A, la peau reconstruite est cultivée en absence de vitamine C et pour les clichés B, la peau reconstruite est cultivée en présence de vitamine C. Pour les clichés de gauche, la peau a reçu deux types de coloration, l'une ciblant les noyaux des cellules (sphéroïdes sur la partie haute des photographies), l'autre mettant en évidence la présence de collagène VII (ligne horizontale). Dans les deux clichés de droite, seule la coloration mettant en évidence la présence de collagène VII est représentée.

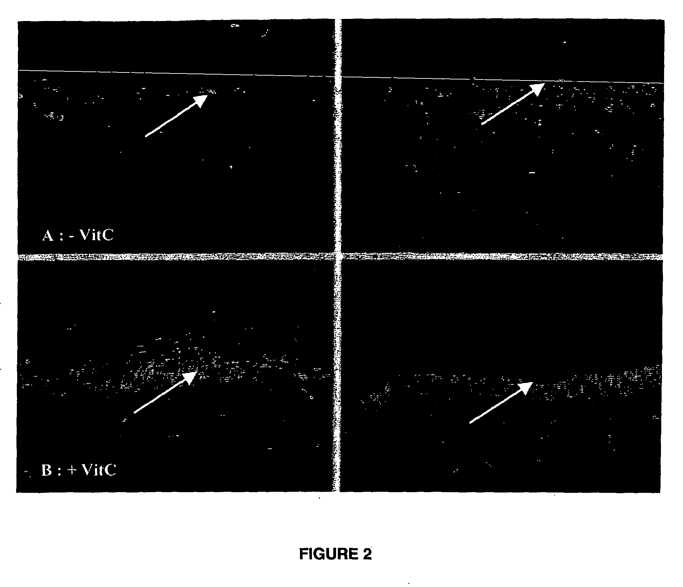

La **figure 2** représente des photographies de coupe transversale de peau reconstruite : pour les clichés A, la peau reconstruite est cultivée en absence de vitamine C et pour les clichés B, la peau reconstruite est cultivée en présence de vitamine C. Pour les clichés de gauche, la peau a reçu deux types de coloration, l'une ciblant les noyaux des cellules (sphéroïdes sur la partie haute des photographies), l'autre mettant en évidence la présence de ténascine (ligne horizontale). Dans les deux clichés de droite, seule la coloration mettant en évidence la présence de ténascine est représentée.

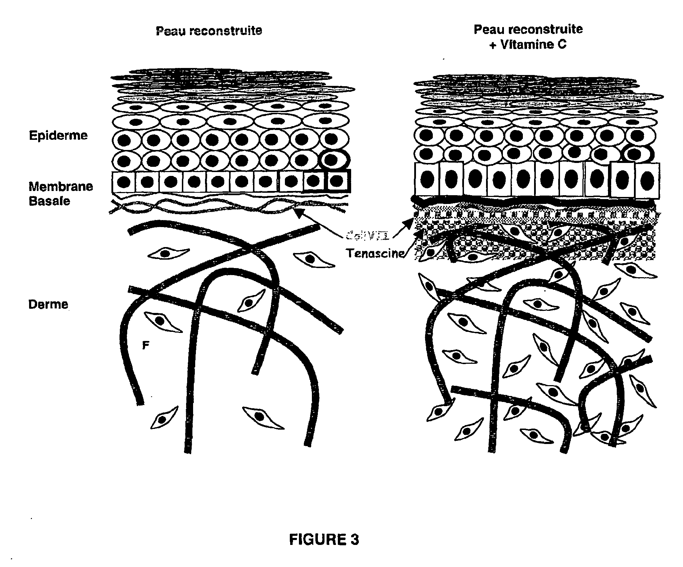

La **figure 3** est une représentation schématique d'une coupe transversale de peau reconstruite, la partie gauche du schéma représente une peau reconstruite cultivée sans vitamine C, la partie de droite représente une peau reconstruite cultivée en présence de vitamine C.

### EXEMPLES

Les exemples suivants illustrent l'invention sans en limiter aucunement la portée. Les pourcentages sont donnés en poids par rapport au poids total de la composition.

### Exemple 1 : Gel à la vitamine C tamponné pH = 4

- **Gel :**
   - Phase A
      - Carraghénane 1,5 %
      - Lubrajel 28,5 %
      - Conservateur 0,2 %
      - Eau permutée qsp 95 %
      - Soude 0,5 %
   - Phase B
      - Eau permutée 5,3 %
      - Germall 115 0,3 %
      - Dequest 2046 0,1 %
- **Solide :**
   - Acide ascorbique 5 %

Le gel est fabriqué de façon classique en préparant, séparément, par simple mélange les phases A et B respectivement à 70 °C et 40 °C, puis en ajoutant la phase B à la phase A sous agitation à 40 °C et en laissant refroidir l'ensemble sous agitation lente jusqu'à la température ambiante.

### Exemple 2 : Emulsion eau-dans-huile

### Phase huileuse :

| | |
|---|---|
| Diméthicone copolyol et cyclométhicone (« 3225C Formulation Aid » vendu par Dow Corning) | 8 % |
| Phényltriméthylsiloxytrisiloxane (« 556 Fluid » vendu par Dow Corning) | 15 % |
| Tocophérol | 0,5 % |

### Phase aqueuse :

| | |
|---|---|
| Propylène glycol | 39,4 % |
| Polyéthylène glycol 400 | 13 % |
| Sel disodique de l'acide éthylène diamine tétracétique (agent chélateur) | 0,1 % |
| Acide ascorbique | 3 % |
| Eau | 21% |

Le mode opératoire pour préparer l'émulsion est le suivant : on prépare la phase aqueuse d'une part et la phase huileuse d'autre part, et on émulsionne la phase aqueuse dans la phase huileuse à température ambiante sous agitation à l'homogénéisateur.

### Exemple 3 - observation de l'effet de l'adjonction de vitamine C sur la synthèse de la ténascine et du collagène VII sur le modèle de peau reconstruite

Le présent exemple décrit les effets de l'adjonction d'acide ascorbique sur une peau reconstruite par l'observation au microscope de coupe de peau avec un marquage immunohistochimique des protéines de ténascine et collagène VII.

### 1. Préparation de la peau reconstruite

La peau reconstruite utilisée est réalisée selon le protocole décrit dans Asselineau et al. (Models in Dermato. Editions Loire and Maibach, 1987, Vol III,, 1-7). Les modifications à ce protocole sont :
- l'utilisation de fibroblastes de derme humains normaux adultes à raison de 10⁶ cellules par derme équivalent ;
- l'ensemencement des kératinocytes se fait à raison de 50000 cellules par anneau de 1.5 cm de diamètre, les kératinocytes utilisés proviennent d'un même donneur et sont en passage 1 lors de l'ensemencement des équivalents de derme ;
- la durée de la phase d'immersion est de 7 jours ; -
- la durée de la phase d'émersion est de 7 jours.

### 2. Addition de la vitamine C

La molécule utilisée est l'Ascorbyl Phosphate de Magnésium (ci-après vitamine CPMg) utilisée à la concentration finale de 2.8.10⁻⁴ M.

Le dernier changement de milieu de la phase d'immersion est réalisé en présence de vitamine CPMg. La culture est ensuite montée sur grille pour la phase d'émersion (7 jours) et durant cette phase, tous les changements de milieu (tous les 2 jours) sont effectués en présence de vitamine CPMg.

### 3.a. Analyse du collagène VII

L'analyse des peaux reconstruites se fait à la fin de la phase d'émersion. Un échantillon témoin (absence de vitamine CPMg) est systématiquement réalisé et analysé en parallèle.

Les échantillons sont prélevés et congelés en azote liquide. Les blocs sont réalisés en Tissue teck. Le collagène de type VII est détecté par immunohistochimie sur des coupes congelées de 5µm. La technique classique d'immunoflurescence indirecte est réalisée avec un anti-corps monoclonal anti-collagène VII (LH7.2, Chemicon International Inc., USA) et un conjugué couplé à la fluoresceïne (FITC-conjugated Rabbit anti mouse immunoglobulins, DAKO, Denmark).

### 3.b. Analyse de la ténascine

Le protocole utilisé est celui décrit au point 3.a. ci-dessus à la différence que dans ce cas la ténascine est détectée avec un anti-corps monoclonal anti-ténascine (TN2, Chemicon) et un conjugué couplé à la fluoresceïne (FITC-conjugated Rabbit anti mouse immunoglobulins, DAKO, Denmark).

### 4. Observations

### 4.a. collagène VII

En observation microscopique (voir la Figure 1, clichés A : peau reconstruite cultivée en absence de vitamine C et clichés B : peau reconstruite cultivée en présence de vitamine C), la présence de collagène VII se traduit par une fluorescence au niveau de la zone jonctionnelle entre l'épiderme et le derme. On constate que l'intensité et l'épaisseur de la zone de fluorescence, correspondant à la jonction dermo-épidermique, est bien plus importante dans l'échantillon auquel il a été ajouté de la vitamine C.

Les clichés de gauche et de droite de la figure 1 correspondent aux mêmes zones des coupes de peaux. Sur le cliché de gauche, tous les noyaux des cellules ont été colorés à l'iodure de propidium qui permet de se repérer facilement au niveau du tissu. L'épiderme (très cellulaire donc comprenant de nombreux noyaux visualisables par des taches claires de forme circulaire au dessus de la ligne désignée par la flèche) est situé sur la partie haute de la coupe, et le derme (tissu beaucoup moins cellulaire comprenant des fibroblastes distribués dans la matrice extracellulaire) est situé dans la partie basse de la coupe. La zone de fluorescence révélant la présence de collagène VII est localisée au niveau jonctionnel entre l'épiderme et le derme sous forme d'un marquage linéaire (au niveau de la flèche).

La mesure de la zone de fluorescence est réalisée par analyse d'image (Quantimètre Leica Q500W), et normalisée pour les échantillons contrôle et traitée par rapport à une longueur identique de jonction dermo-épidermique (unités arbitraires).

| | | |
|---|---|---|
| Collagène VII | contrôle | + vitamine C |
| Intensité fluorescence /Unité de longueur | 1,35 | 2,50 (X 1,85) |

### 4.b. Ténascine

La présence de ténascine se traduit par une fluorescence au niveau de la zone jonctionnelle entre l'épiderme et le derme qui diffuse dans la partie superficielle du derme. On constate que l'intensité et l'épaisseur de la zone de fluorescence (voir la figure 2, clichés A : peau reconstruite cultivée en absence de vitamine C et clichés B : peau reconstruite cultivée en présence de vitamine C), correspondant à la jonction dermo-épidermique et au derme superficiel, sont bien plus importantes dans l'échantillon auquel il a été ajouté de la vitamine C.

Les clichés de gauche et de droite correspondent aux mêmes zones des coupes de peaux. Sur le cliché de gauche, tous les noyaux des cellules ont été colorés à l'iodure de propidium qui permet de se repérer facilement au niveau du tissu. L'épiderme (très cellulaire donc comprenant de nombreux noyaux visualisables par des taches claires de forme circulaire au dessus de la ligne désignée par la flèche) est situé sur la partie haute de la coupe, et le derme (tissu beaucoup moins cellulaire comprenant des fibroblastes distribués dans la matrice extracellulaire) est situé dans la partie basse de la coupe. La ténascine révélée par immunomarquage fluorescent est distribuée au niveau de la jonction dermo-épdiermique et dans la zone sous épidermique correspondant au derme superficiel (au niveau de la flèche).

La mesure de la zone de fluorescence est réalisée par analyse d'image (Quantimètre Leica Q500W), et normalisée pour les échantillons contrôle et traitée par rapport à une longueur identique de jonction dermo-épidermique (unités arbitraires).

| | | |
|---|---|---|
| ténascine | contrôle | + vitamine C |
| Intensité fluorescence /Unité de longueur | 6.40 | 11.54 (X 1,80) |

Les résultats obtenus montrent donc une amélioration de la jonction dermo-épidermique par la vitamine CPMg, qui est mieux schématisée à la Figure 3 annexée.

## Revendications

1. Utilisation cosmétique de l'acide ascorbique ou l'un de ses analogues dans une composition comprenant un milieu cosmétiquement acceptable pour augmenter la synthèse de collagène VII et la résistance de la peau aux agressions mécaniques choisis parmi frottements, tensions, frictions.

2. Utilisation selon la revendication 1 **caractérisée en ce que** les analogues de l'acide ascorbique sont choisis parmi ses sels, ses esters, et ses sucres.

3. Utilisation selon la revendication 2, **caractérisée en ce que** les analogues de l'acide ascorbique sont choisis parmi l'ascorbate de sodium, l'ascorbylphosphate de magnésium, de sodium, ses esters acétique, propionique, palmitique et l'acide ascorbique glycosylé.

4. Utilisation selon l'une quelconque des revendications précédentes, **caractérisée par le fait que** la quantité d'acide ascorbique ou de ses analogues représente de 0,001 % à 20 %, préférentiellement de 0,1 % à 15 %, et avantageusement de 1 % à 10 % en poids par rapport au poids total de la composition.

## Claims

1. Cosmetic use of ascorbic acid or an analogue thereof in a composition comprising a cosmetically acceptable medium to increase the synthesis of collagen VII and to increase the resistance of the skin to mechanical attacking factors chosen from rubbing, tension and friction.

2. Use according to Claim 1, **characterized in that** the analogues of ascorbic acid are chosen from its salts, its esters and its sugars.

3. Use according to Claim 2, **characterized in that** the ascorbic acid analogues are chosen from sodium ascorbate, magnesium ascorbyl phosphate, sodium ascorbyl phosphate, its acetic, propionic and palmitic esters, and glycosyl ascorbic acid.

4. Use according to any one of the preceding claims, **characterized in that** the amount of ascorbic acid or of analogues thereof represents from 0.001% to 20%, preferably from 0.1% to 15% and advantageously from 1% to 10% by weight, relative to the total weight of the composition.

## Patentansprüche

1. Kosmetische Verwendung von Ascorbinsäure oder einem ihrer Analoga in einer Zusammensetzung, die ein kosmetisch akzeptables Medium enthält, um die Synthese von Collagen VII zu fördern und die Widerstandskraft der Haut gegenüber mechanischen Einwirkungen zu erhöhen, die unter Reibung, Spannung und Friktion ausgewählt sind.

2. Verwendung nach Anspruch 1, **dadurch gekennzeichnet, dass** die Analoga von Ascorbinsäure unter ihren Salzen, ihren Estern und ihren Zuckern ausgewählt sind.

3. Verwendung nach Anspruch 2, **dadurch gekennzeichnet, dass** die Analoga von Ascorbinsäure unter Natriumascorbat, Magnesiumascorbylphosphat, Natriumascorbylphosphat, ihrem Essigsäureester, Propionsäureester und Palmitinsäureester und glykosylierter Ascorbinsäure ausgewählt sind.

4. Verwendung nach einem der vorgehenden Ansprüche, **dadurch gekennzeichnet, dass** der Mengenanteil der Ascorbinsäure oder ihrer Analoga 0,001 bis 20 %, vorzugsweise 0,1 bis 15 % und vorteilhaft 1 bis 10 Gew.-%, bezogen auf das Gesamtgewicht der Zusammensetzung, ausmacht.
